# EUROPEAN PATENT SPECIFICATION

(11) **EP 1 771 161 B1**
(45) Date of publication and mention of the grant of the patent: **21.04.2010**
(21) Application number: 05759832.8
(22) Date of filing: 11.07.2005
(51) Int. Cl.: A61K 9/22, A61K 31/495

(54) **TASTE MASKING FORMULATION COMPRISING THE DRUG IN A DISSOLUTION-RETARDED FORM AND/OR CYCLODEXTRIN IN A DISSOLUTION-ENHANCED FORM**
GESCHMACKSABDECKENDE FORMULIERUNG MIT EINER RETARDIERTEN WIRKSTOFFFORMULIERUNG UND/ODER SCHNELL LÖSLICHEM CYCLODEXTRIN
FORMULATION DE MASQUAGE DU GOUT COMPRENANT UN MEDICAMENT A DISSOLUTION RETARDEE ET/OU DE LA CYCLODEXTRINE A DISSOLUTION AMELIOREE

(30) Priority: 22.07.2004 US 590803 P
(43) Date of publication of application: 11.04.2007
(73) Proprietor: Bend Research, Inc, Bend OR 97701-8599 (US)
(72) Inventor: Chu, Jennifer Hsing-Chung, Eastern Point Road, Groton, CT 06340 (US); Friesen, Dwayne Thomas, Bend, OR 97701 (US); Lyon, David Keith, Bend, OR 97701 (US); Ketner, Rodney James, Bend, OR 97701 (US)
(74) Representative: Church, Simon John
(86) International application number: PCT/IB2005/002231
(87) International publication number: WO 2006/011051

(56) References cited:
- WO-A-03/084511
- US-A- 5 206 025
- US-A1- 2002 032 217
- US-A1- 2004 115 258

## Description

### BACKGROUND OF THE INVENTION

The present invention relates to pharmaceutical compositions comprising cyclodextrins that provide taste masking for unpleasant tasting drugs.

Cyclodextrins are cyclic multicyclopyranose units connected by alpha-(1,4) linkages. The most widely known cyclodextrins are alpha, beta and gamma-cyclodextrins. Derivatives of these cyclodextrins are also known and used in the pharmaceutical field. The cyclic nature of the cyclodextrins, the hydrophobic properties of their cavities as well as the hydrophilic character of their outer surfaces, enables them to interact with other chemicals and to produce inclusion compounds.

Numerous reviews and patents related to the use of cyclodextrins and their derivatives to prepare inclusion complexes of drugs are found in the literature, for example, D. Duchene, Cyclodextrins and their Industrial Uses, Editions de Sante, Paris, 1987, Chapter 6 (211-257), Chapter 8 (297-350), Chapter 10 (393-439); D. Duchene et al, Acta Pharma Technol. 36(1)6, 1-6, 1990; D. Duchene et al, Drug Dev. Ind. Pharm., 16(17), 2487-2499, 1990; C. Hunter et al, European Patent Publication No. EP 0346006, December 1988.

Inclusion complexes prepared to specifically improve water solubility and hence bioavailability of poorly soluble drugs have been reported by workers such as D. D. Chow et al, Int. J. Pharm., 28, 95-101, 1986; F. A. Menard et al, Drug Dev. Ind. Pharm., 14(11), 1529-1547, 1988; F. J. Otera-Espinar et al, Int. J. Pharm., 75, 37-44, 1991; and Berand M. Markarian et al, European Patent Publication No. EP 0274444, July 1988. Chemical modifications of cyclodextrins to prepare derivatives that further improve solubility of water insoluble drugs have been described, for example, by J. Pitha, U.S. Pat. No. 4,727,064, February 1988; N. S. Bodor, U.S. Pat. No. 5,024,998, July 1991.

Cyclodextrins have also been used to taste mask unpleasant tasting drugs, for example in US2004/115258. Cyclodextrins form complexes with the drug in an aqueous environment that limit or reduce contact of uncomplexed drug with the taste buds; often these complexes have improved taste over the uncomplexed drug.

Nevertheless, taste masking with cyclodextrins is not always successful. For some unpleasant tasting drugs, a blend of the cyclodextrin and drug does not provide adequate taste masking. For example, a blend of cetirizine and beta-cyclodextrin still results in the bitter taste of cetirizine being tasted almost immediately.

Fanarra, US 2002/0032217 A1, discloses pre-forming the drug:cyclodextrin complex and subsequently incorporating the pre-formed complex into dosage forms. Fanarra discloses forming solutions of cetirizine and beta-cyclodextrin that had reduced bitter taste due to the pre-formation of a drug:cyclodextrin complex. However, an inherent drawback of this approach is that incorporation of a pre-formed drug:cyclodextrin complex into a dosage form requires that the complex be prepared, isolated and purified.

There is therefore still a need for an effective method of taste masking unpleasant tasting drugs that does not require pre-formation of the drug:cyclodextrin complex, and that overcomes the drawbacks of physical mixtures of cyclodextrin and drug. These needs are met by the present invention, which is summarized and described in detail below.

### BRIEF SUMMARY OF THE INVENTION

In a first aspect, a pharmaceutical composition comprises a physical mixture of an unpleasant-tasting drug in solid form and a cyclodextrin in solid form wherein the drug is in a dissolution-retarded form selected from a multiparticulate and a coating. By "physical mixture" is meant one wherein substantially no complexation has taken place between the drug and the cyclodextrin in the composition.

In a second aspect, a pharmaceutical composition comprises a physical mixture of an unpleasant-tasting drug in solid form and a cyclodextrin in solid form wherein the cyclodextrin is in a dissolution-enhanced form selected from an amorphous form and a mean particle size less than about 125µm.

In a third aspect, a pharmaceutical composition comprises a physical mixture of an unpleasant-tasting drug in solid form and a cyclodextrin in solid form wherein the drug is in a dissolution-retarded form selected from a multiparticulate and a coating and the cyclodextrin is in a dissolution-enhanced form selected from an amorphous form and a mean particle size less than about 125µm.

The present invention overcomes the drawbacks of the prior art by allowing cyclodextrins to be used to provide taste masking for a wider variety of drugs. The inventors found that a problem when using cyclodextrins to provide taste masking was that for some drugs, the drug dissolved too quickly in the mouth, allowing the drug to be tasted before the cyclodextrin complex formed. This resulted in an initial unpleasant taste that slowly subsided as the complex formed.

As a result of this discovery, it has been found that improved taste masking of drugs with cyclodextrins results when a molar excess of dissolved cyclodextrin is maintained relative to the number of moles of dissolved drug in the buccal use environment. This molar excess of cyclodextrin in solution is achieved by manipulating the dissolution rates of the drug and the cyclodextrin. More specifically, it has been found that taste masking is improved by either (i) retarding the drug dissolution rate or (ii) enhancing the cyclodextrin dissolution rate or (iii) both (i) and (ii). While not wishing to be bound by any theory, such manipulation of the forms of the drug and cyclodextrin so as to alter their respective dissolution rates provides effective *in situ* formation of complexes between the drug and cyclodextrin in a buccal *(in vivo)* use environment. Preferably, the ratio of (1) the molar concentration of dissolved cyclodextrin in the buccal use environment to (2) the molar concentration of dissolved drug during the first minute after administration is at least about 1, more preferably at least about 1.5, even more preferably at least about 2, and most preferably at least about 3. This ratio is maintained by retarding the drug dissolution rate or by enhancing the cyclodextrin dissolution rate or by both, by one or more of the methods disclosed herein. By maintaining a molar excess of dissolved cyclodextrin relative to dissolved drug, the cyclodextrin may complex more effectively with the dissolved drug so as to limit the concentration of dissolved drug in the mouth relative to that obtained when the molar ratio of dissolved cyclodextrin to dissolved drug is less. This allows the composition to provide superior taste masking of unpleasant tasting drugs.

As used herein, a "use environment" refers to either *in vivo* fluids, such as present in the buccal space or the GI tract of an animal, such as a mammal, and particularly a human, or to the *in vitro* environment of a test solution, such as a simulated mouth buffer (MB) or a simulated gastric buffer (GB). An appropriate simulated MB test solution is 0.05M KH₂PO₄ buffer adjusted to pH 7.3 with 10M KOH. Appropriate GB test solutions include 0.01N HCl and 0.1N HCl. "Administration" to a use environment means, where the *in vivo* use environment is the mouth or GI tract placing the composition in the mouth, ingestion or other such means to deliver the composition. Where the use environment is *in vitro,* "administration" refers to placement or delivery of the composition or dosage form containing the composition to the *in vitro* test medium.

The foregoing and other objectives, features, and advantages of the invention will be more readily understood upon consideration of the following detailed description of the invention.

### DETAILED DESCRIPTION OF PREFERRED EMBODIMENTS

In order to provide taste masking, the pharmaceutical compositions of the present invention control the dissolution rate of at least one of the drug and cyclodextrin, or both. In general, it is preferred for the dissolution rates to be controlled, so that a molar excess of dissolved cyclodextrin relative to dissolved drug is present in the buccal use environment. The molar excess of dissolved cyclodextrin allows the drug:cyclodextrin complex to form rapidly as drug dissolves. This limits the concentration of dissolved drug in the buccal use environment, and thus provides taste masking of the unpleasant tasting drug. Drug and cyclodextrin dissolution rates, relative amounts of cyclodextrin and drug, methods for retarding the dissolution rate of the drug, methods for increasing the dissolution rate of cyclodextrin, and exemplary dosage forms are described in more detail below.

### Drugs

The invention is applicable to any unpleasant tasting drug that has some degree of solubility in water. The invention finds particularly desirable application in the case of unpleasant tasting drugs that are fast-dissolving. By "fast-dissolving" is meant a drug in its crystalline form that is at least 20% dissolved within about one minute in an *in vitro* simulated mouth buffer solution when administered in an amount such that, if all of the drug dissolved, the concentration of dissolved drug would be 30% of the solubility of the drug. A suitable *in vitro* simulated mouth buffer solution is 0.05M KH₂PO₄ at pH 7.3. By retarding the dissolution rate of the drug, the composition allows the cyclodextrin sufficient time to dissolve so as to be available in solution to complex with the drug as the drug dissolves. Thus, the invention finds increasing utility when the drug has even faster dissolution rates, such as at least 50% dissolved within about one minute, or even at least 70% dissolved within about one minute. The invention also finds utility in the case of slow dissolving drugs that have low taste thresholds; that is, drugs that may be tasted at low dissolved drug concentrations.

Exemplary drugs that may be used with the current invention include, without limitation, inorganic and organic compounds that act on the peripheral nerves, adrenergic receptors, cholinergic receptors, nervous system, skeletal muscles, cardiovascular smooth muscles, blood circulatory system, synaptic sites, neuroeffector junctional sites, endocrine and hormone systems, immunological system, reproductive system, autocoid systems, alimentary and excretary systems, inhibitors of autocoids and histamine systems. Preferred classes of drugs include, but are not limited to, antacids, analgesics, anti-anginals, anti-anxiety agents, anti-arrhythmics, anti-bacterials, antibiotics, anti-diarrheals, anti-depressants, anti-epileptics, anti-fungals, anti-histamines, anti-hypertensives, anti-inflammatory agents, anti-virals, cardiac agents, contraceptives, cough suppressants, cytotoxics, decongestants, diuretics, drugs for genito-urinary disorders, drugs for use in parkinsonism and related disorders, drugs for use in rheumatic disorders, hypnotics, minerals and vitamins, lipid lowering drugs and sex hormones. Veterinary drugs may also be suitable for use with the present invention.

Each named drug should be understood to include the neutral form of the drug and pharmaceutically acceptable forms thereof. By "pharmaceutically acceptable forms" thereof is meant any pharmaceutically acceptable derivative or variation, including stereoisomers, stereoisomer mixtures, enantiomers, solvates, hydrates, isomorphs, polymorphs, pseudomorphs, salt forms and prodrugs.

Specific examples of unpleasant-tasting drugs include acetaminophen, albuterol, aminoguanidine hydrochloride, aminophylline, amitriptyline, amoxicillin trihydrate, ampicillin, amlodipine besylate, aspirin, azithromycin, barbiturates, berberine chloride, caffeine, calcium carbonate, calcium pantothenate, cephalosporins, cetirizine, chloramphenicol, chlordiazepoxide, chloroquine, chlorpheniramine, chlorpromazine, cimetidine, ciprofloxacin, clarithromycin, codeine, demerol, dextromethorphan, digitoxin, digoxin, diltiazem hydrochloride, diphenhydramine, diphenylhydantoin, doxazosin mesylate, doxylamine succinate , eletriptan, enoxacin, epinephrine, erythromycin, ethylefrine hydrochloride, etinidine, famotidine, fluconazole, glipizide, guaifenesin, ibuprofen, indeloxazine hydrochloride, lidocaine, lomotil, loratadine, lupitidine, magnesium oxide, meclizine, methacholine, morphine, neostigmine, nifentidine, niperotidine, nizatidine, ofloxacin, paracetamol, pefloxacin, penicillin, phenobarbital, phenothiazine, phenylbutazone, phenylpropanolamine, pipemidic acid, pirbuterol hydrochloride, piroxicam , prednisolone, propranolol hydrochloride, pseudoephedrine, pyridonecarboxylic acid antibacterials, ranitidine, roxatidine, salicylic acid, sertaraline hydrochloride, sildenafil, spironolactone, sulbactam sodium, sulfonamides, sulfotidine, sulpyrine, sultamicillin tosylate , tenidap, terfenadine, theophylline, trimethoprim, tuvatidine, valdecoxib, zaltidine, and zonisamide.

### Cyclodextrin

Cyclodextrins useful in the present invention include α-, β- and γ-cyclodextrins and alkyl and hydroxyalkyl derivatives thereof, with β-cyclodextrins and derivatives of β-cyclodextrin being the most preferred from the standpoint of availability and cost. Exemplary derivatives of cyclodextrin include mono- or polyalkylated β-cyclodextrin, mono- or polyhydroxyalkylated β-cyclodextrin, mono, tetra or hepta-substituted β-cyclodextrin, and sulfoalkyl ether cyclodextrin (SAE-CD). Specific cyclodextrin derivatives for use herein include hydroxypropyl-β-cyclodextrin, hydroxyethyl-β-cyclodextrin, hydroxypropyl-γ-cyclodextrin, hydroxyethyl-γ-cyclodextrin, dihydroxypropyl-β-cyclodextrin, glucosyl-α-cyclodextrin, glucosyl-β-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, sulfobutyl ether cyclodextrin (SBE-CD), and mixtures thereof such as maltosyl-β-cyclodextrin/dimaltosyl-β-cyclodextrin. A preferred cyclodextrin is β-cyclodextrin.

### Dissolution Rates

In one embodiment, the drug is in a dissolution-retarded form. By a "dissolution-retarded" form of the drug is meant a form of the drug that has a dissolution rate that is slower than that of the crystalline form of the drug. More specifically, when the dissolution-retarded drug form is placed in a use environment, such as the *in vivo* buccal use environment or an *in vitro* simulated mouth buffer solution, the amount of dissolved drug provided by the dissolution-retarded forms measured after about one minute is less than 90% of the amount of dissolved drug provided by a control composition consisting essentially of an equivalent amount of the crystalline drug alone measured after one minute in the same mouth buffer solution. The control composition is simply the initial crystalline form of the drug prior to preparation of the dissolution-retarded form. Preferably, the dissolution-retarded form slows the dissolution of the drug so that the amount of dissolved drug in the mouth buffer solution after about one minute is less than 80% of that provided by crystalline drug, even more preferably less than 70%, and most preferably less than 60% of the control.

A suitable *in vitro* dissolution test to evaluate whether the drug is in a dissolution retarded form may be performed as follows. An amount of drug in dissolution-retarded form is placed in an aqueous buffer solution comprising 0.05M KH₂PO₄ adjusted to pH 7.3 with 10M KOH, at a concentration such that if all of the drug dissolved, the concentration would be less than 30% of the solubility of the drug in the aqueous buffer solution. This solution is equilibrated to 37°C and stirred at a constant speed. After one minute, a sample is removed and filtered or centrifuged to remove undissolved particles. The sample is analyzed to determine the concentration of drug dissolved from the dissolution-retarded form. The procedure is repeated in a separate buffer solution with a control composition consisting of an equivalent amount of bulk crystalline drug, and the amount of crystalline drug dissolved in one minute is determined. The ratio of the amount of dissolved drug provided by the dissolution-retarded form to amount of dissolved drug provided by the control composition is calculated and expressed as a percent. For example, if the amount of dissolved drug provided by the dissolution-retarded form about one minute after administration was 40 wt% of the total amount of drug in the dissolution retarded-form administered to the buffer solution, and the amount of dissolved drug provided by the control composition after about one minute was 80 wt% of the total amount of drug in the control composition, then the amount of dissolved drug provided by the dissolution-retarded form was 50% of the amount of dissolved drug provided by the control composition.

However, while the dissolution-retarded form slows the rate of dissolution of the drug relative to bulk crystalline drug, nonetheless the compositions also provide substantially immediate release of the drug in the Gl tract. Thus, the compositions release at least about 70 wt%, preferably at least about 80 wt%, and more preferably at least about 90 wt% of the drug within one hour following administration to a use environment. Compositions may be tested in an appropriate buffered or non-buffered aqueous solution, such as simulated MB test solution of 0.05M KH₂PO₄ buffer adjusted to pH 7.3 with 10M KOH, or in a simulated GB test solution, to determine whether they meet the release criteria described above. The amount of the composition tested relative to the amount of buffer solution is such that the ratio of the amount of drug to the volume of buffer solution is less than 30% of the drug solubility in the buffer solution.

In a separate embodiment, the cyclodextrin is in a dissolution-enhanced form. By a "dissolution-enhanced form" of cyclodextrin is meant cyclodextrin in a form that dissolves more rapidly than the most common commercially available forms of cyclodextrin, i.e., crystalline cyclodextrin having a volume weighted mean particle diameter of from 150 to 350 µm. More specifically, when the dissolution-enhanced form of cyclodextrin is placed in a use environment, such as the *in vivo* buccal use environment or an *in vitro* simulated mouth buffer solution, the amount of cyclodextrin dissolved during the first minutes in the use environment is greater than the amount dissolved provided by a control composition consisting essentially of crystalline cyclodextrin having a volume weighted mean particle diameter of about 150 µm. Preferably, the dissolution-enhanced form increases the amount of the cyclodextrin that is dissolved during the first minute is greater than about 1.25-fold, even more preferably greater than about 1.5-fold, and most preferably greater than about 2-fold that provided by the control composition. The inventors determined that the amount of dissolved cyclodextrin provided by crystalline cyclodextrin having a volume weighted mean particle diameter of about 150 µm was about 41%. Accordingly, the dissolution enhanced form preferably is at least about 50%, even more preferably at least about 60%, and most preferably at least about 80% dissolved one minute after administration to the use environment.

A suitable *in vitro* dissolution test to measure the amount of dissolved cyclodextrin is as follows. An amount of cyclodextrin in dissolution-enhanced form is placed in an aqueous simulated mouth buffer solution consisting of 0.05M KH₂PO₄ adjusted to pH 7.3 with 10M KOH, at a concentration such that if all of the cyclodextrin dissolved, the concentration of dissolved cyclodextrin would be less than 30% of the solubility of the cyclodextrin in the aqueous buffer solution. This solution is stirred at 37°C. Samples are removed one minute after administration and filtered or centrifuged to remove undissolved particles. The samples are analyzed to determine the concentration of cyclodextrin dissolved from the dissolution-enhanced form, and the amount of dissolved cyclodextrin is calculated. The procedure is repeated using a control composition consisting of an equivalent amount of bulk crystalline cyclodextrin having a volume weighted mean particle diameter of 150 µm.

### Relative Amounts

Effective *in situ* complexation of the cyclodextrin to the drug is accomplished when the molar concentration of dissolved cyclodextrin is greater than the molar concentration of dissolved active drug in a buccal use environment. In terms of a molar ratio of cyclodextrin dissolved to drug dissolved in the same buffer solution noted above, the molar ratio of cyclodextrin:drug measured after 1 minute is preferably greater than about 1, more preferably at least about 1.5, even more preferably at least about 2.0, and most preferably at least about 3. For example, using the same in vitro dissolution test described above in connection with dissolution rates, if after one minute the concentration of dissolved drug was 1 x 10⁻³ mmol/ml, then the dissolved cyclodextrin concentration is preferably at least 1 x 10⁻³ mmol/ml, more preferably at least 1.5 x 10⁻³ mmol/ml, even more preferably at least 2 x 10⁻³ mmol/ml, and most preferably at least 3 x 10⁻³ mmol/ml.

The cyclodextrin is thus present in the compositions in a sufficient amount so as to provide the desired molar ratio of dissolved cyclodextrin to dissolved drug. This may be determined by determining the molar concentration of dissolved drug provided by a composition one minute after administration to an *in vitro* simulated mouth buffer, determining the dissolution rate of the cyclodextrin, and then calculating the amount of cyclodextrin needed to achieve a molar concentration of dissolved cydodextrin that is at least as great as the molar concentration of dissolved drug. For example, beta-cyclodextrin has a molecular weight of 1,134 mg/mmol. If a composition when administered to 900 ml of the *in vitro* simulated mouth buffer provides a molar concentration of dissolved active drug of 1.5 x 10⁻⁵ mmol/ml after one minute, and the amount of beta-cyclodextrin dissolved after one minute is 50 wt% of the beta-cyclodextrin originally present in the composition, then the composition should contain at least 31 mg of beta-cyclodextrin to achieve a molar ratio of dissolved cyclodextrin:drug measured after one minute that is greater than 1 (1.5 x 10⁻⁵ mmol/ml x 900 ml x 1,134 mg/mmol/50%).

One of the advantages of the compositions of the invention is that at short times after administration (e.g., 0.1 to 2 minutes) the amount of cyclodextrin needed to achieve effective taste masking is reduced relative to compositions that do not contain either a drug in a dissolution-retarded form or cyclodextrin in a dissolution-enhanced form. The compositions achieve a higher molar ratio of dissolved cyclodextrin:drug for the same amount of cyclodextrin and drug relative to a conventional composition in which the dissolution rate of neither the drug or cyclodextrin is modified. Accordingly, for a given amount of drug to be taste masked, less cyclodextrin is needed to achieve taste masking relative to a conventional composition.

### Methods for Retarding Dissolution of Drug

Methods for forming dissolution-retarded forms of the drug include (i) forming multiparticulates of the drug (including particles, granules, etc.) ; and (ii) coating the drug, either alone or in a multiparticulate formulation.

Drug-containing multiparticulates may be formed by any conventional method, such as by a melt-congeal method, granulation (both dry and wet) and extrusion-spheronization. The multiparticulates may be coated or uncoated. Multiparticulates are small, having a mean diameter of up to about 3 mm. (Reference to diameter is to the diameter of the finished multiparticulate, including the coating, if present.) A useful measure of their size that takes into account diameter and volume frequency is volume-weighted mean diameter. The volume-weighted mean assumes a gaussian size distribution, with approximately 85% of the particle volume being within about 30% of the reported size. The inventive multiparticulates preferably have a volume-weighted mean diameter of less than 500 microns, and more preferably less than about 300 microns. While such multiparticulates can have any shape and texture, it is preferred that they be spherical, with a smooth surface texture. These sizes and shapes lead to excellent flow properties, ease of compaction into tablets, improved "mouth feel," ease of swallowing and ease of uniform coating, if desired.

In a preferred embodiment, the multiparticulates are very small. Preferably, such multiparticulates have a volume-weighted mean diameter (after coating, if present) of less than 200 microns, and more preferably less than about 150 microns. For coated multiparticulates, the uncoated core has a volume-weighted mean diameter of less than 150 microns, more preferably less than about 125 microns, and even more preferably less than 100 microns. Such small multiparticulates are more pleasing to patients, since such small multiparticulates present a smooth, rather than gritty sensation in the mouth, if such multiparticulates are even felt at all. In addition, such small multiparticulates are particularly advantageous when incorporating coated multiparticulates into a chewable tablet. The inventors have discovered that one problem associated with forming compressed chewable tablets using coated multiparticulates is that the coating may break during formation of the tablet. Such dosage forms often contain hard crystalline material, such as microcrystalline cellulose, saccharides such as sucrose or xylitol, or polyols like mannitol or sorbitol. It is believed that the compression of such hard crystalline materials into the multiparticulates causes the coating to break or fracture. In addition, the coatings of large multiparticulates may break or fracture during chewing. However, the inventors have found that small multiparticulates are much less likely to experience broken coatings during compression or chewing.

One method of forming the drug into multiparticulates is by a melt-congeal process, which comprises the steps of (a) forming a molten mixture comprising the unpleasant-tasting drug and at least one pharmaceutically compatible carrier, (b) atomizing the molten mixture of step (a) by an atomizing means to form droplets, and (c) congealing the droplets from step (b) to form multiparticulates. By "carrier" is meant all non-drug species present in the multiparticulates, including optional excipient(s).

The molten mixture comprises the drug and at least one pharmaceutically compatible carrier. The drug in the molten mixture may be dissolved in the carrier, may be a suspension of crystalline drug distributed in the molten carrier, or any combination of such states or those states that are in between. Preferably the molten mixture is a homogeneous suspension of crystalline drug in the molten carrier where the fraction of drug that melts or dissolves in the molten carrier is kept relatively low, preferably less than about 30 wt%. Generally, the mixture is molten in the sense that it will flow when subjected to one or more forces such as pressure, shear, and centrifugal force, such as that exerted by a centrifugal or spinning-disk atomizer. Thus, the drug/carrier mixture may be considered "molten" when any portion of the carrier and drug become sufficiently fluid that the mixture, as a whole, is sufficiently fluid that it may be atomized.

Virtually any process may be used to form the molten mixture. One method involves heating the carrier in a tank until it is fluid and then adding the drug to the molten carrier. Generally, the carrier is heated to a temperature of about 10°C or more above the temperature at which it becomes fluid. The process is carried out so that at least a portion of the molten mixture remains fluid until atomized. Once the carrier has become fluid, the drug may be added to the fluid carrier. Alternatively, both the drug and the solid carrier may be added to the tank and the mixture heated until the carrier has become fluid.

An alternative method of preparing the molten mixture is to use two tanks, melting a first carrier in one tank and a second in another. The drug is added to one of these tanks and mixed as described above. The two melts are then pumped through an in-line static mixer or extruder to produce a single molten mixture that is directed to the atomization process described below.

Once the carrier has become fluid and the drug has been added, the mixture is mixed to ensure the drug is substantially uniformly distributed therein. Mixing is generally done using mechanical means, such as overhead mixers, magnetically driven mixers and stir bars, planetary mixers, and homogenizers. Optionally, the contents of the tank can be pumped out of the tank and through an in-line, static mixer or extruder and then returned to the tank. The amount of shear used to mix the molten feed should be sufficiently high to ensure substantially uniform distribution of the drug in the molten mixture. However, it is preferred that the shear not be so high such that the form of the drug is changed, *i.e*., so as to cause a portion of the crystalline drug to become amorphous or to change to a new crystalline form of the drug. Generally, it is preferred to limit the mixing time to near the minimum necessary to disperse the crystalline drug substantially uniformly throughout the molten carrier. Another method that can be used to prepare the molten mixture is to use a continuously stirred tank system. In this system, the drug and carrier are continuously added to a heated tank equipped with means for continuous stirring, while the molten mixture is continuously removed from the tank. The drug is typically added in solid form and may be preheated prior to addition to the tank. The carrier may also be preheated or even pre-melted prior to addition to the continuously stirred tank system. A wide variety of mixing methods can be used with such a system, such as those described above.

The molten mixture may also be formed using a continuous mill, such as a Dyno® Mill wherein solid drug and carrier are fed to the mill's grinding chamber containing grinding media, such as beads with diameters of 0.25 to 5 mm. The grinding chamber typically is jacketed so heating or cooling fluid may be circulated around the chamber to control the temperature in the chamber. The molten mixture is formed in the grinding chamber, and exits the chamber through a separator to remove the grinding media from the molten mixture.

Another method of forming the molten mixture is by an extruder. By "extruder" is meant a device or collection of devices that creates a molten extrudate by heat and/or shear forces and/or produces a uniformly mixed extrudate from a solid and/or liquid (i.e., molten) feed. Such devices include, but are not limited to, single-screw extruders; twin-screw extruders, including co-rotating, counter-rotating, intermeshing, and non-intermeshing extruders; multiple screw extruders; ram extruders, consisting of a heated cylinder and a piston for extruding the molten feed; and gear-pump extruders, consisting of a heated gear pump, generally counter-rotating, that simultaneously heats and pumps the molten feed; and conveyer extruders. Conveyer extruders comprise a conveyer means for transporting solid and/or powdered feeds, such as a screw conveyer or pneumatic conveyer, and a pump. At least a portion of the conveyer means is heated to a sufficiently high temperature to produce the molten mixture. The molten mixture may optionally be directed to an accumulation tank, before being directed to a pump, which directs the molten mixture to an atomizer. Optionally, an in-line mixer may be used before or after the pump to ensure the molten mixture is substantially homogeneous. In each of these extruders the molten mixture is mixed to form a uniformly mixed extrudate. Such mixing may be accomplished by various mechanical and processing means, including mixing elements, kneading elements, and shear mixing by backflow. Thus, in such devices, the composition is fed to the extruder, which produces a molten mixture that can be directed to the atomizer.

In one embodiment, the composition is fed to the extruder in the form of a solid powder. The powdered feed can be prepared using methods well known in the art for obtaining powdered mixtures with high content uniformity. See Remington's Pharmaceutical Sciences (20th ed. 2000). Generally, it is desirable that the particle sizes of the drug and carrier be similar to obtain a uniform blend. However, this is not essential to the successful practice of the invention.

Once the molten mixture has been formed, it is delivered to an atomizer that breaks it into small droplets. Virtually any method can be used to deliver the molten mixture to the atomizer, including the use of pumps and various types of pneumatic devices such as pressurized vessels or piston pots. When an extruder is used to form the molten mixture, the extruder itself can be used to deliver the molten mixture to the atomizer. Typically, the molten mixture is maintained at an elevated temperature while delivering the mixture to the atomizer to prevent solidification of the mixture and to keep the molten mixture flowing.

Generally, atomization occurs in one of several ways, including (1) by "pressure" or single-fluid nozzles; (2) by two-fluid nozzles; (3) by ultrasonic nozzles; (4) by mechanical vibrating nozzles; and (5) by centrifugal or spinning-disk atomizers. Detailed descriptions of atomization processes can be found in Lefebvre, Atomization and Sprays (1989) or in Perry's Chemical Engineers' Handbook (7th Ed. 1997), the disclosures of which are incorporated herein by reference.

There are many types and designs of pressure nozzles, which generally deliver the molten mixture at high pressure to an orifice. The molten mixture exits the orifice as a filament or as a thin sheet that breaks up into filaments, which subsequently break up into droplets. The operating pressure drop across the pressure nozzle ranges from 1 barg to 70 barg, depending on the viscosity of the molten feed, the size of the orifice, and the desired size of the multiparticulates.

In two-fluid nozzles, the molten mixture is contacted with a stream of gas, typically air or nitrogen, flowing at a velocity sufficient to atomize the molten mixture. In internal-mixing configurations, the molten mixture and gas mix inside the nozzle before discharging through the nozzle orifice. In external-mixing configurations, high velocity gas outside the nozzle contacts the molten mixture. The pressure drop of gas across such two-fluid nozzles typically ranges from 0.5 barg to 10 barg.

In ultrasonic nozzles, the molten mixture is fed through or over a transducer and horn, which vibrates at ultrasonic frequencies, atomizing the molten mixture into small droplets. In mechanical vibrating nozzles, the molten mixture is fed through a needle vibrating at a controlled frequency, atomizing the molten mixture into small droplets. In both cases, the particle size produced is determined by the liquid flow rate, frequency of ultrasound or vibration, and the orifice diameter.

In centrifugal atomizers, also known as rotary atomizers or spinning-disk atomizers, the molten mixture is fed onto a rotating surface, where it is caused to spread out by centrifugal force. The rotating surface may take several forms, examples of which include a flat disk, a cup, a vaned disk, and a slotted wheel. The surface of the disk may also be heated to aid in formation of the multiparticulates. Several mechanisms of atomization are observed with flat-disk and cup centrifugal atomizers, depending on the flow of molten mixture to the disk, the rotation speed of the disk, the diameter of the disk, the viscosity of the feed, and the surface tension and density of the feed. At low flow rates, the molten mixture spreads out across the surface of the disk and when it reaches the edge of the disk, forms a discrete droplet, which is then flung from the disk. As the flow of molten mixture to the disk increases, the mixture tends to leave the disk as a filament, rather than as a discrete droplet. The filament subsequently breaks up into droplets of fairly uniform size. At even higher flow rates, the molten mixture leaves the disk edge as a thin continuous sheet, which subsequently disintegrates into irregularly sized filaments and droplets. The diameter of the rotating surface generally ranges from 2 cm to 50 cm, and the rotation speeds range from 500 rpm to 100,000 rpm or higher, depending on the desired size of the multiparticulates.

Once the molten mixture has been atomized, the droplets are congealed, typically by contact with a gas or liquid at a temperature below the solidification temperature of the droplets. The congealing step often occurs in an enclosed space to simplify collection of the multiparticulates. In such cases, a cooling gas or liquid may be circulated through the enclosed space to maintain a relatively constant congealing temperature.

Multiparticulates may also be formed by dry-granulating or wet-granulating the drug with one or more of the pharmaceutically compatible carriers disclosed herein. Dry granulation may be effected by milling the drug and carrier, such as by ball mills, hammer mills, fluid energy mills or roller compactors; by blending the drug and carrier with mixers such as planetary mixers, vortex blenders and V-blenders; and by mixing the drug and carrier in extruders, such as a twin-screw extruder.

Wet granulation may be effected in high-shear granulators or in fluid bed granulators wherein a solvent or wetting agent is added to the ingredients, or a carrier material may be dissolved in a solvent that is used as a granulating fluid.

When the multiparticulate is made using a mixing or blending process, the granulation process can occur in the same equipment used to form the blend, such as conventional high-shear or high speed mixers/granulators that are routinely used to process pharmaceutical compositions. In these processes, a granulation fluid is mixed with the composition after the dry components have been blended to aid in the formation of the granulated composition. Examples of granulation fluids include water, ethanol, isopropyl alcohol, n-propanol, the various isomers of butanol and mixtures thereof.

If a wet granulation process is used, the granulated drug and carrier or carriers are often dried prior to further processing. Examples of suitable drying processes to be used in connection with wet granulation are tray drying, microwave drying, rotary drying and fluid bed drying, all well known in the pharmaceutical arts. Once the drug has been granulated, it may then be milled to achieve the particle size desired for the specific dosage form.

Multiparticulates may also be formed by another form of wet granulation known in the pharmaceutical arts as "extrusion/sperhonization." In this process, the drug and carrier material are mixed with a liquid to form a paste-like plastic suspension, which is then extruded through a perforated plate or die to form a solid mass, often in the form of elongated, solid rods. This solid mass is then milled to form the multiparticulates. In one embodiment, the solid mass is placed, with or without an intervening drying step, onto a rotating disk that has protrusions that break the material into multiparticulate spheres, spheroids, or rounded rods. The so-formed multiparticulates are then dried to remove any remaining liquid.

The drug-containing multiparticulates formed by any of the methods described above include a pharmaceutically acceptable carrier, which in general refers to all non-drug species or excipients present in the multiparticulates. By "pharmaceutically acceptable" is meant the carrier must be compatible with the other ingredients of the composition, and not be deleterious to the patient. The carrier functions as a matrix for the multiparticulate or to control the rate of release of drug from the multiparticulate, or both. For some drugs, a single carrier may sufficiently delay drug release, while for other drugs, two or more carriers may be required to provide the desired dissolution-retarded form of the drug.

The carrier comprises at least a matrix materials. Exemplary matrix materials include cellulosic polymers such as microcrystalline cellulose, ethyl cellulose, methyl cellulose, hydroxyethyl cellulose, hydroxypropyl cellulose, hydroxypropylmethyl cellulose, carboxymethyl cellulose, carboxyethyl cellulose, carboxymethyl ethyl cellulose, cellulose acetate, cellulose acetate phthalate, cellulose acetate butyrate, hydroxypropylmethyl cellulose phthalate and hydroxypropylmethyl cellulose acetate succinate; styrene and maleic acid copolymers; polyacrylic acid derivatives such as acrylic acid and acrylic ester copolymers; crotonic acid copolymers; highly purified forms of waxes, such as Carnauba wax, white and yellow beeswax, microcrystalline wax, and paraffin wax; long-chain alcohols, such as stearyl alcohol, cetyl alcohol and polyethylene glycol; poloxamers; polyoxyethylene alkyl ethers; long-chain fatty acid esters (also known as fats), such as glyceryl monooleate, glyceryl monostearate, glyceryl palmitostearate, polyethoxylated castor oil derivatives, mixtures of mono-, di-, and trialkyl glycerides, including mixtures of glyceryl mono-, di-, and tribehenate, glyceryl tristearate, glyceryl tripalmitate and hydrogenated vegetable oils, including hydrogenated cottonseed oil; glycolized fatty acid esters, such as polyethylene glycol stearate and polyethylene glycol distearate; short to medium chain fatty acid esters, such as isopropyl palmitate, isopropyl myristate, triethyl citrate, lecithin, triacetin, and dibutyl sebacate; polysorbates; carboxylic acids such as stearic acid, benzoic acid, citric acid, fumaric acid, lactic acid, and maleic acid; and mixtures thereof. Especially preferred matrix materials are an alkyl-containing glycerol such as a mixture of mono-, di- and triglyceryl behenates (commercially available as COMPRITOL 888 from Gattefose Corporation of Westwood, New Jersey); hydrogenated cottonseed oil (commercially available as LUBRITAB from Edward Mendell Co. of Patterson, New York). The matrix material may comprise mixtures of materials, such as mixtures of any of the foregoing.

The carrier may also include one or more optional excipient(s). One particularly useful class of excipients that may be included in the carrier comprises dissolution-inhibiting agents, which can be used to inhibit or delay the release of the drug from the multiparticulates or coated drug particles. Such dissolution-inhibiting agents are generally hydrophobic. Examples of dissolution-inhibiting agents include: hydrocarbon waxes, such as microcrystalline and paraffin wax; and polyethylene glycols having molecular weights greater than about 20,000 daltons.

Although the aim of the invention is generally to retard the drug dissolution rate, in some cases it may be necessary to adjust that rate upwardly to achieve fast release of the drug after leaving the mouth. In such cases a very useful class of excipients comprises dissolution-enhancing agents, which increase the rate of dissolution of the drug from the multiparticulates or coated drug particle. Such agents may make up 0 to 30 wt % of the multiparticulate or coated drug particle, based on the total mass of the same. In general, dissolution enhancers are amphiphilic compounds and are generally more hydrophilic than the carrier. Exemplary dissolution enhancers include alcohols such as stearyl alcohol, cetyl alcohol, and polyethylene glycol; dispersing or emulsifying agents, such as poloxamers, docusate salts, polyoxyethylene alkyl ethers, polyoxyethylene castor oil derivatives, polysorbates, polyoxyethylene alkyl esters, sodium lauryl sulfate, and sorbitan monoesters; sugars such as glucose, sucrose, xylitol, sorbitol, and maltitol; salts such as sodium chloride, potassium chloride, lithium chloride, calcium chloride, magnesium chloride, sodium sulfate, potassium sulfate, sodium carbonate, magnesium sulfate, and potassium phosphate; amino acids such as alanine and glycine; and mixtures thereof.

When drug multiparticulates are formed by a melt-congeal process, a useful class of excipients comprises materials used to adjust the viscosity of the molten feed. Such viscosity-adjusting excipients will generally make up 0 to 65 wt% of the multiparticulate, based on the total mass of the multiparticulate. The viscosity of the molten feed is a key variable in obtaining multiparticulates with a narrow particle size distribution. For example, when a spinning-disk atomizer is employed, it is preferred that the viscosity of the molten mixture be at least about 1 cp and less than about 10,000 cp, more preferably at least 50 cp and less than about 1000 cp. If the molten mixture has a viscosity outside these preferred ranges, a viscosity-adjusting carrier can be added to obtain a molten mixture within the preferred viscosity range. Examples of viscosity-reducing excipients include stearyl alcohol, cetyl alcohol, low molecular weight polyethylene glycol (*e.g.,* less than about 1000 daltons), isopropyl alcohol, and water. Examples of viscosity-increasing excipients include microcrystalline wax, paraffin wax, synthetic wax, high molecular weight polyethylene glycols (*e.g.*, greater than about 5000 daltons), ethyl cellulose, hydroxypropyl cellulose, hydroxypropyl methyl cellulose, methyl cellulose, silicon dioxide, microcrystalline cellulose, magnesium silicate, sugars, and salts.

Other excipients may be added to adjust the release characteristics of the multiparticulates or coated drug particles or to improve processing and will typically make up 0 to 50 wt% of the multiparticulate or coated drug particle, based on the total mass of the same. For example, excipients may be added to reduce the static charge on the multiparticulates or coated drug particles. Examples of such anti-static agents include talc and silicon dioxide. Flavorants, colorants, and other excipients may also be added in their usual amounts for their usual purposes.

An exemplary multiparticulate formulation comprises 5 to 80 wt% drug and 20 to 95 wt% of a carrier. In one embodiment the multiparticulate comprises 10 to 55 wt% drug; 90 to 45 wt% of a carrier selected from waxes, such as synthetic wax, microcrystalline wax, paraffin wax, Carnauba wax, and beeswax; glycerides, such as glyceryl monooleate, glyceryl monostearate, glyceryl palmitostearate, polyethoxylated castor oil derivatives, hydrogenated vegetable oils, glyceryl mono-, di- or tribehenates, glyceryl tristearate, glyceryl tripalmitate and mixtures thereof; and 0 to 30 wt% of a dissolution-enhancing excipient selected from dispersing or emulsifying agents, such as poloxamers, polyoxyethylene alkyl ethers, polyethylene glycol, polysorbates, polyoxyethylene alkyl esters, sodium lauryl sulfate, and sorbitan monoesters; alcohols, such as stearyl alcohol, cetyl alcohol and polyethylene glycol; sugars, such as glucose, xylitol, sorbitol and maltitol; salts, such as sodium chloride, potassium chloride, lithium chloride, calcium chloride, magnesium chloride, sodium sulfate, potassium sulfate, sodium carbonate, magnesium sulfate and potassium phosphate; amino acids, such as alanine and glycine; and mixtures thereof. Although it is desirable to deliver the drug in a dissolution-retarded form relative to crystalline drug, it is nevertheless desirable to release at least 70% of the drug within an hour of dosing. Therefore, in some formulations a dissolution-enhancing excipient may be needed as described above.

In another exemplary embodiment of a drug-containing multiparticulate, the multiparticulate comprises (a) drug; (b) a glyceride carrier having at least one alkylate substituent of 16 or more carbon atoms; and (c) a poloxamer. Two particularly preferred glyceride carriers comprise *(i)* a mixture of glyceryl mono- di- and tribehenates, commercially available as COMPRITOL 888 from Gattefosse Corporation of Westwood, New Jersey, and *(ii)* hydrogenated cottonseed oil, commercially available as LUBRITAB from Edward Mendell Co. of Patterson, New York.

Dissolution-retarded forms may also be made by coating any of the drug-containing multiparticulates described above, or by coating particles of drug alone with one or more of the pharmaceutically compatible carriers disclosed herein, using standard coating equipment, such as pan coaters (e.g., Hi-Coater available from Freund Corp. of Tokyo, Japan, Accela-Cota available from Manesty of Liverpool, U.K.), fluidized bed coaters (*e.g.*, Würster coaters or top-spray coaters, available from Glatt Air Technologies, Inc. of Ramsey, New Jersey and from Niro Pharma Systems of Bubendorf, Switzerland), rotary granulators (*e.g.,* CF-Granulator, available from Freund Corp), and spray driers (*e.g*., Niro type XP Portable Spray-Dryer with a Liquid Feed Process Vessel Model No. PSD-1).

In one method, especially when using a liquid-based or latex-based process for forming the coating, a Würster fluidized-bed system is used. In this system, a cylindrical partition (the Würster column) is placed inside a conical product container in the apparatus. Air passes through a distribution plate located at the bottom of the product container to fluidize the drug particles, with the majority of the upward moving air passing through the Würster column. The drug particles are drawn into the Würster column, which is equipped with an atomizing nozzle that sprays the coating solution upward. The drug particles are coated as they pass through the Würster column, with the coating solvent being removed as the coated particles exit the column.

Alternatively, a top-spray method can be used to apply the coating. In this method, coating solution is sprayed down onto the fluidized drug particles. The solvent evaporates from the coated particles, which are re-fluidized in the apparatus. Coating is continued until the desired coating thickness is achieved.

The coating may also be applied using a hot-melt coating technique. In this method, the coating is first melted and then sprayed onto the drug particles. Typically, the hot-melt coating is applied in a fluidized bed equipped with a top-spray arrangement.

Another method for applying a hot-melt coating to drug particles is to use a modified melt-congeal method. In this method, the drug particles are suspended in the molten coating, the melting point of the drug particles being greater than the melting point of the coating. This suspension is then formed into droplets comprising the drug particles surrounded by the coating. The droplets are typically formed through the use of an atomizer, such as a rotary or spinning-disk atomizer. The droplets are then cooled to congeal the coating, forming the coated drug particles.

The coating may also be applied in a rotary granulator. In such devices, horizontal disks rotate at high speed, forming a rotating "rope" of drug particles at the walls of the vessel. The coating is sprayed into this rope, coating the particles. This technique can be used with hot-melt, latex, and liquid-based coating solutions.

Preferred coating materials include cellulose ethers such as ethyl cellulose, methyl cellulose, hydroxyethyl cellulose, hydroxypropyl cellulose, and hydroxypropylmethyl cellulose, carboxymethyl cellulose, carboxyethyl cellulose, carboxymethyl ethyl cellulose; cellulose esters, such as cellulose acetate, cellulose acetate propionate, cellulose acetate butyrate, cellulose acetate phthalate; styrene and maleic acid copolymers; polyacrylic acid derivatives such as acrylic acid and acrylic ester copolymers; crotonic acid copolymers, polymethacrylates, polyethylene glycol, polyethylene oxide, polypropylene glycol, polyethylene-polypropylene glycol copolymers, polyvinyl pyrrolidinone, starch, dextran, dextrin, polydextrose, polyalkenes, polyethers, polysulfones, polyethersulfones, polystyrenes, polyvinyl alcohol, polyvinyl halides, polyvinyl ethers, paraffin wax, microcrystalline wax, Carnauba wax, synthetic wax, acacia, gelatin, beeswax, gums, shellac and sugar. A particularly preferred cellulose ether is ethyl cellulose (commercially available as SURELEASE from Colorcon of West Point, Pennsylvania). A particularly preferred polymethacylate is a 2:1 copolymer of ethyl acrylate and methyl methacrylate (commercially available as EUDRAGIT NE from Röhm Pharma of Darmstadt, Germany).

The coating may include conventional plasticizers, including dibutyl phthalate; dibutyl sebacate; diethyl phthalate; dimethyl phthalate; triethyl citrate; benzyl benzoate; butyl and glycol esters of fatty acids; refined mineral oils; oleic acid; stearic acid; cetyl alcohol; stearyl alcohol; castor oil; corn oil; coconut oil; and camphor oil; and other excipients such as anti-tack agents, glidants, etc. For plasticizers, triethyl citrate, coconut oil and dibutyl sebacate are particularly preferred.

### Dissolution Enhanced Forms of Cyclodextrin

As described above, dissolution-enhanced forms of cyclodextrin have a dissolution rate that is faster than commercial grades of cyclodextrin (e.g., cyclodextrin having a volume weighted mean particle diameter of about 150 microns). Dissolution-enhanced forms of cyclodextrin include (i) cyclodextrin having a volume weighted mean particle diameter of less than about 150 microns; and (ii) amorphous cyclodextrin.

A particularly preferred and simple method for forming a dissolution-enhanced form of cyclodextrin involves breaking larger diameter particles into smaller diameter particles. In general, dissolution rate increases as the mean particle size decreases. A preferred smaller mean diameter particle size range is less than 125 µm, more preferably less than 100 µm, and even more preferably less than 75 µm. Particle size reduction may be accomplished by any conventional method, such as by milling, grinding, micronizing, atomization, and precipitation. Exemplary milling devices include a chaser mill, ball mill, vibrating ball mill, hammer mill, impact grinding mill, fluid energy mill (jet mill), and centrifugal-impact pulverizers. A preferred method is jet milling. Cyclodextrin with particle sizes of less than about 150 µm can also be formed by other means, such as dissolution in a solvent such as alcohol or water followed by precipitation by mixing with a non-solvent. Another method to reduce particle size is by atomization, such as by dissolving the cyclodextrin in a solvent and atomizing the resulting solution by spray drying to form a powder.

Another method for forming a dissolution-enhanced form of cyclodextrin is to convert crystalline cyclodextrin to the amorphous form. The amorphous form may be made by any conventional method. For example, the amorphous form may be made by dissolving the cyclodextrin in a matrix and solidifying the matrix and cyclodextrin rapidly to prevent recrystallization of the cyclodextrin; by adsorbing amorphous cyclodextrin onto a porous substrate; by dissolving cyclodextrin in a solvent and rapidly removing the solvent from the solution to obtain the amorphous cyclodextrin alone; or by incorporating cyclodextrin into a solid amorphous dispersion of cyclodextrin in a fast-dissolving water-soluble pharmaceutically compatible matrix. Suitable techniques for preparing such amorphous forms of cyclodextrin include melt-congealing or spray-congealing a molten mixture of the cyclodextrin and a carrier, or spray-drying or lyophilizing a solution of the cyclodextrin alone or with a carrier, porous substrate, or matrix.

Porous substrates useful for forming adsorbates of amorphous cyclodextrin include inorganic oxides such as SiO₂, TiO₂, ZnO₂, ZnO, Al₂O₃, and zeolites.

The components used in the matrix may be polymeric or non-polymeric, and may comprise a mixture of several components. Thus the matrix may comprise a mixture of polymeric components, a mixture of non-polymeric components, or a mixture of polymeric and non-polymeric components.

The term "polymeric" is used conventionally, meaning a compound that is made of monomers connected together to form a larger molecule. A polymeric component generally consists of at least about 20 monomers. Thus, the molecular weight of a polymeric component will generally be about 2000 daltons or more. The polymeric component may be neutral or ionizable, and may be cellulosic or non-cellulosic. In general, polymers useful in forming solid amorphous dispersions of cyclodextrin may be neutral or ionizable cellulosic or non-cellulosic amphiphilic polymers with an aqueous-solubility of at least 0.1 mg/mL. Examples of neutral non-cellulosic polymers include vinyl polymers and copolymers, polyvinyl alcohols, polyvinyl alcohol/ polyvinyl acetate copolymers, polyethylene glycol/ polypropylene glycol copolymers, polyvinyl pyrrolidone, polyethylene/ polyvinyl alcohol copolymers, and polyoxyethylene/ polyoxypropylene block copolymers. Examples of ionizable non-cellulosic polymers include carboxylic acid functionalized polymethacrylates and carboxylic acid functionalized polyacrylates, amine-functionalized polyacrylates and polymethacrylates, high molecular weight proteins such as gelatin and albumin. and carboxylic acid functionalized starches such as starch glycolate. Examples of neutral cellulosic polymers are hydroxypropyl methyl cellulose, hydroxyethyl cellulose, hydroxypropyl cellulose, methyl cellulose, hydroxyethyl methyl cellulose, and hydroxyethyl ethyl cellulose. Examples of ionizable cellulosic polymers are hydroxypropyl methyl cellulose acetate succinate, hydroxypropyl methyl cellulose phthalate, carboxymethyl ethyl cellulose, carboxyethyl cellulose, carboxymethyl cellulose, cellulose acetate phthalate, hydroxypropyl methyl cellulose acetate phthalate, and cellulose acetate trimellitate.

By "non-polymeric" is meant that the component is not polymeric. Exemplary non-polymeric materials for use as a matrix component include: alcohols, such as stearyl alcohol and cetyl alcohol, organic acids and their salts, such as stearic acid, citric acid, fumaric acid, tartaric acid, malic acid, and pharmaceutically acceptable salts thereof; organic bases such as glucosamine, N-methylglucamine, tris (hydroxymethyl)amino methane, and dodecylamine; salts such as sodium chloride, potassium chloride, lithium chloride, calcium chloride, magnesium chloride, sodium sulfate, potassium sulfate, sodium carbonate, and magnesium sulfate; amino acids such as alanine and glycine; sugars such as glucose, sucrose, xylitol, fructose, lactose, trehalose, mannitol, sorbitol, and maltitol; fatty acid esters such as glyceryl (mono- and di-) stearates, glyceryl (mono- and di-) behenates, triglycerides, sorbitan monostearate, saccharose monostearate, glyceryl (palmitic stearic) ester, hydrogenated cottonseed oil, polyoxyethylene sorbitan fatty-acid esters; waxes, such as microcrystalline wax, paraffin wax, beeswax, synthetic wax, castor wax, and carnauba wax; alkyl sulfates such as sodium lauryl sulfate and magnesium lauryl sulfate; and phospholipids, such as lecithin; and mixtures thereof.

For some matrix materials which have low water solubility or which have a tendency to gel in aqueous solution, it may be necessary to form a porous material to obtain a fast dissolution rate of the amorphous cyclodextrin. For example, for the matrix hydroxypropyl methyl cellulose, the amorphous cyclodextrin may be formed by lyophilization to obtain a highly porous material that dissolves rapidly.

### Excipients and Dosage Forms

Although the key ingredients present in the compositions of the present invention are simply the dissolution-retarded form of the drug and the dissolution-enhanced form of cyclodextrin, the inclusion of other excipients in the composition may be useful.

Conventional formulation excipients may be employed in the compositions of this invention, including those excipients well-known in the art (*e.g*., as described in Remington's Pharmaceutical Sciences (20th ed. 2000)). Generally, excipients such as fillers, disintegrating agents, pigments, binders, lubricants, glidants, flavorants, and so forth may be used for customary purposes and in typical amounts without adversely affecting the properties of the compositions. These excipients may be utilized after the drug and cyclodextrin compositions have been formed, in order to formulate the compositions into the desired dosage form. The addition of pH modifiers such as acids, bases, or buffers may also be beneficial, modifying the rate of dissolution of the drug or cyclodextrin compositions, or, alternatively, helping to improve the chemical stability of the compositions. Chewable tablets may be formulated using conventional tabletting excipients such as diluents, swelling agents, anti-tack agents, binders, lubricants, flavorings and sweeteners.

In one embodiment the dosage form includes one or more additional tastemasking agents. Examples of additional taste masking agents include sweeteners such as aspartame, compressible sugar, dextrates, lactose, mannitol, sucrose, maltose, sodium saccharin, sorbitol, and xylitol, and flavors such as banana, grape, vanilla, cherry, eucalyptus oil, menthol, orange, peppermint oil, raspberry, strawberry, and watermelon.

Examples of dosage form excipients, fillers, or diluents include lactose, mannitol, xylitol, dextrose, sucrose, sorbitol, compressible sugar, microcrystalline cellulose, powdered cellulose, starch, pregelatinized starch, dextrates, dextran, dextrin, dextrose, maltodextrin, calcium carbonate, dibasic calcium phosphate, tribasic calcium phosphate, calcium sulfate, magnesium carbonate, magnesium oxide, poloxamers such as polyethylene oxide, and hydroxypropyl methyl cellulose.

Examples of surface active agents include sodium lauryl sulfate and polysorbate 80.

Examples of disintegrants include sodium starch glycolate, sodium carboxymethyl cellulose, calcium carboxymethyl cellulose, croscarmellose sodium, crospovidone (polyvinylpolypyrrolidone), methyl cellulose, microcrystalline cellulose, powdered cellulose, starch, pregelatinized starch, and sodium alginate.

Examples of tablet binders include acacia, alginic acid, carbomer, carboxymethyl cellulose sodium, dextrin, ethylcellulose, gelatin, guar gum, hydrogenatetd vegetable oil, hydroxyethyl cellulose, hydroxypropyl cellulose, hydroxypropyl methyl cellulose, methyl cellulose, liquid glucose, maltodextrin, polymethacrylates, povidone, pregelatinized starch, sodium alginate, starch, sucrose, tragacanth, and zein.

Examples of lubricants include calcium stearate, glyceryl monostearate, glyceryl palmitostearate, hydrogenated vegetable oil, light mineral oil, magnesium stearate, mineral oil, polyethylene glycol, sodium benzoate, sodium lauryl sulfate, sodium stearyl fumarate, stearic acid, talc, and zinc stearate.

Examples of glidants include silicon dioxide, talc and cornstarch.

The composition containing the drug and cyclodextrin may be incorporated into any pharmaceutical formulation that may be tasted, including sublingual tablets, chewable tablets, capsules, or unit dose packets, sometimes referred to in the art as "sachets" or "oral powders for constitution" (OPC); syrups; and suspensions. Solid dosage forms, such as chewable tablets for oral administration, are preferred.

One exemplary chewable tablet may be made as follows. First, a dissolution-retarded form of a drug can be made by forming multiparticulates of the drug using a melt-congeal process. Next, the multiparticulates may be combined with cyclodextrin (either the commercial grade or in a dissolution-enhanced form), a filler such as compressible sugar and microcrystalline cellulose (Avicel PH 101 and Avicel CE), a disintegrant such as Ac-Di-Sol, flavorants, and colorants. These ingredients may be mixed, followed by addition of a lubricant such as magnesium stearate, and then followed by additional mixing. The tablet mixture may be compressed using an F-press and ½" flat-faced beveled edge tooling, resulting in tablets with a hardness of 7 - 9 kP.

In another embodiment, the compositions of the present invention may be co-administered, meaning that a drug-containing composition can be administered separately from, but within the same general time frame as, a cyclodextrin-containing composition. Thus, the drug-containing composition can, for example, be administered in its own dosage form which is taken at approximately the same time as the cyclodextrin-containing composition which is in a separate dosage form. When not administered simultaneously, the cyclodextrin-containing composition is preferably administered prior to the drug-containing composition.

In addition to the above additives or excipients, use of any conventional materials and procedures for preparation of suitable dosage forms using the compositions of this invention known by those skilled in the art are potentially useful.

Other features and embodiments of the invention will become apparent from the following examples which are given for illustration of the invention rather than for limiting its intended scope.

### EXAMPLES

### Multiparticulates 1

Cetirizine was incorporated into multiparticulates to provide a dissolution-retarded form of the drug. (Throughout these examples, the dihydrochloride salt of cetirizine was used, having a molecular weight of 461.8 g/mol and an average particle size of about 5µm). Multiparticulates ("Multiparticulates 1") comprising 35 wt% cetirizine in a carrier of 55 wt% glyceryl mono-, di- and tribehenates (COMPRITOL 888) and 10 wt% of a poloxamer (commercially available as PLURONIC F127 from BASF of Mount Olive, New Jersey) were prepared using the following procedure. First, 2357 g of the COMPRITOL and 429 g of the PLURONIC were added to a sealed, jacketed stainless-steel tank (Malto-Mat-Universal MMU 5, Krieger AGG, Switzerland) equipped with counter-rotating mixing paddles and a homogenizer. Heating fluid at 92°C was circulated through the jacket of the tank. After about 60 minutes, the mixture had melted, having a temperature of about 90°C. The mixture was then mixed at 80 rpm for 60 minutes. Next, 1500 g of the drug cetirizine was added to the melt and homogenized for 5 minutes, resulting in a feed suspension of the cetirizine in the molten components.

The feed suspension was then pumped at a rate of 140 g/min using a gear pump (Zenith Pump, Parker Hannifin Corp, Model C-9000, 2.4 cc/rev) to the center of a 4-inch diameter spinning-disk atomizer rotating at 5800 rpm, the surface of which was heated to 90°C. The particles formed by the spinning-disk atomizer were congealed in ambient air to form a total of 4071 g of multiparticulates.

The cetirizine dissolution rate from the so-formed multiparticulates (Multiparticulates 1) was determined using the following procedure. A 37mg sample of the multiparticulates was placed into a USP Type 2 dissoette flask equipped with Teflon-coated paddles rotating at 50 rpm. The concentration of cetirizine would have been 14 µg/ml if all of the drug had dissolved. The flask contained 900 mL of simulated mouth buffer (0.05 M KH₂PO₄ buffer adjusted to pH 7.3 with KOH) held at 37.0 ± 0.5°C. Samples were taken with 10 µm filters attached to a cannula. A 4-mL sample of the fluid in the flask was drawn and the cannula removed. A 0.45-µm filter was attached to the syringe, 2 mL of sample was returned to the dissolution flask, and 1 mL of sample was filtered into a High Performance Liquid Chromatography (HPLC) vial. The remaining solution in the syringe was drawn from the filter to pull any multiparticulates away from the filter, and returned to the flask. Samples were collected at various time points following addition of the multiparticulates to the flask. The samples were analyzed using HPLC (Hewlett Packard 1100; Mac Mod Analytical Zorbax Stablebond CN (SB-CN) column, 5 µm particles, 15 cm x 4.6 mm i.d.; mobile phase 100 mM KH₂PO₄, pH 6.5 / MeOH (50/50) with 1 g/L sodium octanesulfonate at 1.0 mL/min: absorbance measured at 214 nm with a diode array spectrophotometer). Control 1 consisted of 20 mg of crystalline cetirizine in 200 mL receptor solution.

The amount of dissolved drug was calculated based on the potency assay of the formulation. To measure the potency of the dissolution-retarded drug form, about 40 mg of the formulation (sufficient to obtain a concentration of about 0.1 mg/mL of drug in solution) was weighed and added to a 100 mL volumetric flask. Next, 10 mL acetonitrile was added, and the solution was sonicated for 10 minutes. The flask was filled to volume with the HPLC mobile phase, and sonicated for an additional 10 minutes. The solution was filtered and analyzed to determine the total amount of drug in the formulation. The potency assay of the formulation was used to calculate the amount of drug added for each dissolution test. The amount of drug analyzed in each sample was divided by the total amount of drug added for the test (based on the potency assay), and the results are reported as percent of potency assay. The results of these dissolution tests are given in Table 1. Control 1 consisted of crystalline cetirizine alone.

**Table 1**

| Time (min) | Cetirizine Released (% assay) | |
|---|---|---|
| | Multiparticulates 1 | Control 1 |
| 0 | 0 | 0 |
| 1 | 41 | 93 |
| 2 | 47 | 97 |
| 3 | 60 | 97 |
| 5 | 75 | 97 |
| 10 | 89 | 97 |

As is apparent from the data in Table 1, the incorporation of the drug into the multiparticulate composition retarded the drug's dissolution rate. After 1 minute, only 41 % of the dissolution-retarded form of the drug was dissolved in solution, while 93% of the crystalline drug had dissolved in solution. The dissolution-retarded form provided 41% ö 93% x 100 = 44% of the dissolved drug concentration provided by the control composition after one minute. The data also demonstrated that the so-fabricated cetirizine multiparticulates met the target release profile of at least 70% released in ≤60 minutes.

### Multiparticulates 2

Cetirizine HCl was incorporated into a second multiparticulate formulation ("Multiparticulates 2") to provide a dissolution-retarded form of the drug. Multiparticulates comprising 30 wt% cetirizine in a carrier of 60 wt% hydrogenated cottonseed oil (commercially available as LUBRITAB from Edward Mendell Co. of Patterson, New York) and 10 wt% of the poloxamer PLURONIC F127 were prepared using the following procedure. First, 12 g of the LUBRITAB and 2 g of the PLURONIC were added to a tank and melted, as described for Multiparticulates 1. Drug was preheated and 6g was added with manual stirring. A preheated homogenizer shaft was placed in the solution. The mixture was homogenized for 5 minutes at 5000 rpm, resulting in a feed suspension of the cetirizine in the molten components.

The feed suspension was then pumped at a rate of 140 g/min to the center of a spinning-disk atomizer rotating at 5500 rpm, the surface of which was heated to 90°C. The particles formed by the spinning-disk atomizer were congealed in ambient air to form a total of 15g of multiparticulates.

Multiparticulates 2 were tested as described above for Multiparticulates 1. The data are shown in Table 2. Control 1 is repeated for comparison.

**Table 2**

| Time (min) | Cetirizine Released (% assay) | |
|---|---|---|
| | Multiparticulates 2 | Control 1 |
| 0 | 0 | 0 |
| 1 | 22 | 93 |
| 2 | 28 | 97 |
| 3 | 45 | 97 |
| 5 | 64 | 97 |
| 10 | 69 | 97 |
| 20 | 79 | - |
| 30 | 90 | -- |

As is apparent from the data in Table 2, the incorporation of the drug into the multiparticulate composition retarded the drug's dissolution rate. After 1 minute, only 22% of the dissolution-retarded form of the drug was dissolved in solution, while 93% of the crystalline drug had dissolved in solution. The dissolution-retarded form provided 22% ö 93% x 100 = 24% of the dissolved drug concentration provided by the control composition after one minute. The data also demonstrated that the so-fabricated cetirizine multiparticulates met the target release profile of at least 70% released in ≤60 minutes.

### Dissolution-Enhanced Cyclodextrin

In this example, the amount of dissolved cyclodextrin provided by several different β-cyclodextrin samples with varying ranges of particle size was determined. Samples of β-cyclodextrin with average particle sizes ranging from 116 µm to 249 µm were obtained from Roquette America of Keokuk, Iowa. In addition, a β-cyclodextrin sample with an average particle size range of about 57 µm was prepared by first dissolving the 249-µm commercially available sample in water and then lyophilizing the solution to form a fine powder. Volume weighted mean particle diameter in microns for each β-cyclodextrin sample was determined using a Horiba particle sizer.

The dissolution rates of these samples were determined as follows. A 350 mg sample of β-cyclodextrin of a given particle size was placed into 50 mL of deionized water, and the refractive index was measured at the time points noted in Table 2. Refractive index measurements were compared to a calibration curve of standards with known concentration, and the concentration of dissolved β-cyclodextrin versus time was determined. Results by average particle size are shown in Table 3.

**Table 3**

| Time (min) | β-Cyclodextrin Dissolved (%) | | | | |
|---|---|---|---|---|---|
| | 57 µm | 116 µm | 156 µm | 183 µm | 249 µm |
| 0 | 0 | 0 | 0 | 0 | 0 |
| 1 | 102 | 58 | 41 | 45 | 20 |
| 2 | 102 | 76 | 81 | 52 | 26 |
| 3 | 97 | 84 | 96 | 64 | 42 |
| 4 | 99 | 88 | 92 | 64 | 54 |
| 5 | 100 | Not Determined | 95 | 71 | 67 |

These data demonstrate that as the particle size of the β-cyclodextrin is reduced, the amount of cyclodextrin that is dissolved at one minute following administration to the use environment increases. Cyclodextrin having a mean particle size of 116 µm provided an amount of dissolved cyclodextrin at one minute following administration that was about 1.4-fold that provided by the 156 µm cyclodextrin, while the 57 µm cyclodextrin provided an amount of dissolved cyclodextrin at one minute following administration that was about 2.4-fold that provided by the 156 µm cyclodextrin.

### Amorphous β-Cyclodextrin Dispersion

A dissolution-enhanced form of β-cyclodextrin was made by forming a solid amorphous dispersion of β-cyclodextrin. A solid amorphous dispersion of β cyclodextrin was made by the following procedure. A solution comprising 5 wt% β cyclodextrin and 5 wt% hydroxypropyl methyl cellulose in water was spray-dried using a "mini spray-drier". The solution was pumped into a mini spray-drying apparatus via a Cole Parmer 74900 series rate-controlling syringe pump. The drug/polymer solution was atomized through a Spraying Systems Co. two-fluid nozzle, model no. SU1A using a heated stream of nitrogen (100°C). The spray solution was sprayed into an 11-cm diameter stainless steel chamber. The resulting solid amorphous dispersion was collected on filter paper, dried under vacuum, and stored in a dessicator.

### Example 1

Chewable tablets were made containing a dissolution-retarded form of the drug cetirizine and β-cyclodextrin. The tablets contained 3.6 wt% of Multiparticulates 1, 14.1 wt% and 9.7 wt% of two grades of microcrystalline cellulose (Avicel PH200 and Avicel CE15, respectively from FMC Corporation of Philadelphia, Pennsylvania), 60.8 wt% processed sucrose (commercially available as DiPac from Domino Sugar), 1.3 wt% croscarmellose sodium (commercially available as AcDiSol from FMC Corporation), 10.0 wt% β-cyclodextrin (249 µm average particle size), and 0.5 wt% magnesium stearate. To form the tablets, Multiparticulates 1 and the β-cyclodextrin were mixed in the Turbula blender for 10 minutes. Next, the Avicel PH200 was added to the mixture and mixed for 10 minutes in the Turbula blender, then the Avicel CE15 and AcDiSol were added and blended for 10 minutes, the sucrose was added and blended for 10 minutes, and finally magnesium stearate was added and blended for 4 minutes. The mixture was then weighed into 800 mg samples and formed into tablets on an F-Press using 1/2" flat, beveled (FB) tooling. The compression force was set to deliver tablets with a hardness of 7 to 9 kiloponds (kP).

For Control 2, tablets were made in which the drug was not in a dissolution-retarded form. The tablets of Control 2 contained 1.3 wt% crystalline cetirizine, 14.5 wt% Avicel PH200, 10.0 wt% Avicel CE15, 62.5 wt% DiPac, 1.3 wt% AcDiSol, 10.0 wt% β-cydodextrin (249 µm average particle size), and 0.5 wt% magnesium stearate.

### Cetirizine Dissolution Rate

The rate of dissolution of cetirizine from the so-prepared tablets was determined as described above. Each tablet was crushed lightly with a mortar and pestle before it was added to the dissolution flask, to simulate chewing the tablet. The results are shown in Table 4. Cetirizine release from Multiparticulates 1 is shown again for comparison.

**Table 4**

| Time (min) | Cetirizine Released (% assay) | | |
|---|---|---|---|
| | Multiparticulates 1 | Example 1 | Control 2 |
| 0 | 0 | 0 | 0 |
| 1 | 41 | 46 | 77 |
| 2 | 47 | 68 | 86 |
| 3 | 60 | 78 | 88 |
| 5 | 75 | 85 | 90 |
| 10 | 89 | 91 | 93 |
| 20 | -- | 94 | 96 |
| 30 | - | 94 | 96 |

The results show that the initial cetirizine dissolution rate from the tabletted multiparticulates is approximately the same as that from multiparticulates alone and is slower than release of cetirizine from the tabletted crystalline drug (Control 2). The tablet of Example 1 containing the dissolution retarded form of cetirizine provided 60% of the amount of dissolved drug after one minute compared with the tablets of Control 2 containing bulk crystalline cetirizine (46% ö 77% x 100). Nevertheless, the tablet of Example 1 released 94 wt% of the drug after 30 minutes.

The tablet of Example 1 also maintained a dissolved cyclodextrin ratio in excess of dissolved drug during the first minute. The tablet contained 10.1 mg cetirizine. The concentration of cetirizine if all of the drug had dissolved would have been 11 µg/mL (10.1 mg/ 900 ml). The molecular weight of cetirizine is 461.8 mg/mmol. After 1 minute, 46 wt% of the drug had dissolved in the buffer solution. The molar concentration of dissolved cetirizine in solution was therefore 46% x 11 µg/mL x 1 mmol/(461.8 x 10³ µg) = 1.1 x 10⁻⁵ mmoles/mL. The tablet also contained 80 mg β-cyclodextrin. The concentration of β-cyclodextrin in the buffer solution, if all of the cyclodextrin had dissolved, would have been 88.9 µg/mL. The molecular weight of β-cyclodextrin is about 1,134 mg/mmol. It was assumed that 20 wt% of the cyclodextrin was dissolved after one minute (See Table 3). The molar concentration of β-cydodextrin was 20% x 88.9 µg/mL x 1 mmol/(1,134 x 10³µg) = 1.6 x 10⁻⁵ mmoles/mL. The tablet thus provided a molar ratio of dissolved cyclodextrin:dissolved drug after one minute of 1.6 x 10⁻⁵ mmoles/mU 1.1 x 10⁻⁵ mmoles/mL = 1.5. Thus the tablet provided an excess molar ratio of dissolved cyclodextrin to drug after one minute.

### Example 2

Chewable tablets were made as in Example 1 for human taste tests; the tablets contained 3.6 wt% of Multiparticulates 1, 14.9 wt% Avicel PH101, 10.0 wt% Avicel CE15, 58.8 wt% DiPac, 1.1 wt% AcDiSol, 10.0 wt% β-cyclodextrin (249 µm average particle size), 0.3 wt% grape flavor, 0.1 wt% vanilla flavor, 0.1 wt% carmine red colorant, 0.1 wt% FD&C blue #2 lake, and 1.0 wt% magnesium stearate. For Control 3, tablets were made with the same ingredients in the same amounts except that there was 24.9 wt% of the Avicel PH101 and no β-cyclodextrin present. For Control 4, tablets were made with the same ingredients in the same amounts, except the cetirizine was in crystalline form and present at 1.3 wt% and there was 17.2 wt% Avicel PH101. Control 5 comprised cetirizine multiparticulates without any β-cyclodextrin.

Six panelists were given these four formulations in tablet and multiparticulate forms, each containing a dose of 10 mg cetirizine. The taste-test protocol consisted of chewing the formulation until it would normally be swallowed, then holding it in the mouth until bitterness was detected. The degree of bitterness was noted by each participant for each formulation. The results are shown in Table 5.

**Table 5**

| | | | |
|---|---|---|---|
| Example 2 tablet with multiparticulates and β-cyclodextrin | Control 3 tablet with multiparticulates and no β-cyclodextrin | Control 4 tablet with crystalline cetirizine and β-cyclodextrin | Control 5 Cetirizine multiparticulates alone without β-cyclodextrin |
| no bitter taste for 5 of 6 panelists - 1 reported slight bitterness after 45 seconds | immediate bitter taste that grows with time | immediate bitter taste; bitter taste slowly goes away | Slowly building bitter taste |

These results show that the tablets of Example 2, containing cetirizine multiparticulates and β-cyclodextrin, provided a satisfactory taste-masked dosage form.

### Example 3

Chewable tablets were made with the same formulation noted above for Control 2 in Example 1, but the β-cyclodextrin was in the form of 57-µm particles so as to enhance its dissolution rate.

The expected molar ratio of dissolved cyclodextrin to dissolved drug after one minute was calculated as follows. After one minute, the cyclodextrin was expected to be completely dissolved (See Table 3). Assuming a 900 ml simulated mouth buffer solution, the expected molar concentration of dissolved cyclodextrin was 100% x 88.9 µg/mL x 1mmol/(1,134 x 10³ µg) = 7.8 x 10⁻⁵ mmoles/mL. The amount of dissolved cetirizine after one minute was expected to be 77% (see Table 4). The molar concentration of dissolved cetirizine in solution was therefore 77% x 11 µg/mL x 1 mmol/(461.8 x 10³ µg) = 1.8 x 10⁻⁵ mmoles/mL. The expected molar ratio of dissolved cyclodextrin to dissolved cetirizine after one minute was therefore 4.3.

### Example 4

Chewable tablets were made with the same formulation noted above for Control 2 in Example 1, with the following exceptions: the cetirizine was granulated with 2 wt% magnesium stearate before incorporation into the tablet so as to retard its dissolution rate, and only 13.0 wt% of the Avicel PH200 was used.

The tablets of Examples 3 and 4 were taste-tested by the same six panelists using the same protocol as in Example 2. The results are shown in Table 6.

**Table 6**

| | |
|---|---|
| Example 3 | improved taste relative to Controls 3-5 with very faint bitter taste |
| Example 4 | improved taste relative to Controls 3-5 with faint bitter taste |

### Example 5

This example demonstrates calculation of the amount of cyclodextrin required to achieve a desired molar ratio of dissolved cyclodextrin to dissolved drug. A tablet is to be prepared containing 28.9 mg of Multiparticulates 1. The amount of dissolved drug in 900 ml of buffer solution after one minute is assumed to be the same as the amount of dissolved drug provided by the multiparticulates alone, or 41wt% (See Table 4). Thus, the molar concentration of dissolved drug in 900 ml of buffer solution after one minute is 41% x 11 µg/mL x 1mmol/(461.8 x 10³ µg) = 1.0 x 10⁻⁵ mmoles/mL.

In order to achieve a molar ratio of dissolved cyclodextrin to dissolved drug of at least 1.5 after one minute, then the molar concentration of dissolved cyclodextrin after one minute should be at least 1.5 x 1.0 x 10⁻⁵ mmoles/mL = 1.5 x 10⁻⁵ mmoles/mL.

To achieve this molar concentration for the commercial grade of β-cyclodextrin having an average particle size of 249 µm, it is assumed that 20 wt% of the cyclodextrin is dissolved after one minute (See Table 3). The tablet should therefore provide a concentration of dissolved β-cyclodextrin after one minute, if 20 wt% of the cyclodextrin dissolved, of 1.5 x 10⁻⁵ mmoles/mL/20% x 1,134 x 10³µg/mmol = 85.1 µg/mL. Using the same 900 ml volume of buffer solution used in the dissolution tests to achieve the above concentration of dissolved cetirizine, the tablet should contain at least 77 mg of the commercial grade of β-cyclodextrin to achieve the desired molar ratio of 1.5 of dissolved cyclodextrin to dissolved drug after one minute.

Similarly, the amount of cyclodextrin required to achieve various molar ratios of dissolved cyclodextrin to dissolved drug for a composition containing is calculated as set forth in Table 7:

**Table 7**

| Molar ratio of dissolved cyclodextrin to dissolved drug after one minute | Amount of cyclodextrin required |
|---|---|
| 1.5 | 77 mg |
| 2 | 102 mg |
| 3 | 154 mg |

### Example 6

Example 5 is repeated, except that the β-cyclodextrin is milled to a size of 156 µm and is assumed to be 41 % dissolved after one minute (See Table 3). The amount of cyclodextrin required to achieve various molar ratios of dissolved cyclodextrin to dissolved drug for a composition containing 28.9 mg Multiparticulates 1 and β-cyclodextrin milled to a size of 156 µm is calculated as set forth in Table 8:

**Table 8**

| Molar ratio of dissolved cyclodextrin to dissolved drug after one minute | Amount of cyclodextrin required |
|---|---|
| 1.5 | 37 mg |
| 2 | 50 mg |
| 3 | 75 mg |

### Example 7

Example 5 is repeated, except that Multiparticulates 2 and amorphous β-cyclodextrin are used. The dissolution rate of the amorphous β-cyclodextrin is determined in 900 mL of simulated mouth buffer (0.05 M KH₂PO₄ buffer adjusted to pH 7.3 with KOH). The amount of amorphous β-cyclodextrin to be used is calculated using the procedures described above.

The terms and expressions which have been employed in the foregoing specification are used therein as terms of description and not of limitation, and there is no intention in the use of such terms and expressions of excluding equivalents of the features shown and described or portions thereof, it being recognized that the scope of the invention is defined and limited only by the claims which follow.

## Claims

1. A pharmaceutical composition comprising a physical mixture of
(a) an unpleasant-tasting drug in solid form, and
(b) a cyclodextrin in solid form,
wherein said drug is in a dissolution-retarded form selected from a multiparticulate and a coating.

2. A pharmaceutical composition comprising a physical mixture of
(a) an unpleasant-tasting drug in solid form, and
(b) a cyclodextrin in solid form,
wherein said cyclodextrin is in a dissolution-enhanced form selected from an amorphous form and a mean particle size less than 125 µm.

3. A pharmaceutical composition comprising a physical mixture of
(a) an unpleasant-tasting drug in solid form, and
(b) a cyclodextrin in solid form,
wherein said drug is in a dissolution-retarded form selected from a multiparticulate and a coating and said cyclodextrin is in a dissolution-enhanced form selected from an amorphous form and a mean particle size less than 125 µm.

4. The composition of claim 1 or 3 wherein said multiparticulate has a mean diameter of less than 500 µm.

5. The composition of claim 1 or 3 wherein said multiparticulate has a mean diameter of less than 200 µm.

6. The composition of any of claims 1-3 wherein said composition provides within the first minute following administration to a use environment a molar ratio of dissolved cyclodextrin to dissolved drug that is greater than one.

7. The composition of claim 6 wherein said molar ratio of said dissolved cyclodextrin to said dissolved drug is at least 1.5.

8. The composition of claim 6 wherein said molar ratio of said dissolved cyclodextrin to said dissolved drug is at least 2.

9. The composition of claim 6 wherein said molar ratio of said dissolved cyclodextrin to said dissolved drug is at least 3.

10. The composition of claim 1 or 3 wherein said dissolution-retarded form provides a dissolved drug concentration about one minute after administration to a use environment that is less than 80% of the concentration of dissolved drug provided by a control composition consisting of crystalline drug.

11. The composition of claim 2 or 3 wherein said dissolution-enhanced form of said cyclodextrin is at least 50% dissolved at one minute after administration to a use environment.

12. The composition of claim 11 wherein said dissolution-enhanced cyclodextrin is at least 60% dissolved at one minute after administration to the use environment

13. The composition of any one of claims 6, 10 and 11 wherein said use environment is an in vitro test solution comprising a 0.05M KH₂PO₄ buffer solution.

14. The composition of any one of claims 1-3 incorporated into a solid dosage form.

15. The composition of claim 14 wherein said solid dosage form is chewable.

16. The composition of any of claims 1-3 wherein said drug is selected from the group consisting of antacids, analgesics, anti-anginals, anti-anxiety agents, anti-arrhythmics, anti-bacterials, antibiotics, anti-diarrheals, anti-depressants, anti-epileptics, anti-fungals, anti-histamines, anti-hypertensives, anti-inflammatory agents, anti-virals, cardiac agents, contraceptive, cough suppressants, cytotoxics, decongestants, diuretics, drugs for genito-urinary disorders, drugs for use in parkinsonism and related disorders, drugs for use in rheumatic disorders, hypnotics, minerals and vitamins, lipid-lowering drugs and sex hormones

17. The composition of claim 16 wherein said drug is an anti-histamine comprising a benzhydrylpiperazine.

18. The composition of claim 17 wherein said drug is cetirizine.

## Patentansprüche

1. Eine pharmazeutische Zusammensetzung, umfassend ein physikalisches Gemisch von
(a) einem unangenehm schmeckenden Arzneistoff in fester Form, und
(b) einem Cyclodextrin in fester Form,
wobei der Arzneistoff in einer lösungsretardierten Form, ausgewählt aus einer multipartikulären Form und einem Überzug, vorliegt.

2. Eine pharmazeutische Zusammensetzung, umfassend ein physikalisches Gemisch von
(a) einem unangenehm schmeckenden Arzneistoff in fester Form, und
(b) einem Cyclodextrin in fester Form,
wobei das Cyclodextrin in einer lösungsgesteigerten Form, ausgewählt aus einer amorphen Form und einer mittleren Teilchengröße von niedriger als 125 µm, vorliegt.

3. Eine pharmazeutische Zusammensetzung, umfassend ein physikalisches Gemisch von
(a) einem unangenehm schmeckenden Arzneistoff in fester Form, und
(b) einem Cyclodextrin in fester Form,
wobei der Arzneistoff in einer lösungsretardierten Form, ausgewählt aus einer multipartikulären Form und einem Überzug, vorliegt und das Cyclodextrin in einer lösungsgesteigerten Form, ausgewählt aus einer amorphen Form und einer mittleren Teilchengröße von niedriger als 125 µm, vorliegt.

4. Die Zusammensetzung nach Anspruch 1 oder 3, wobei die multipartikuläre Form einen mittleren Durchmesser von weniger als 500 µm aufweist.

5. Die Zusammensetzung nach Anspruch 1 oder 3, wobei die multipartikuläre Form einen mittleren Durchmesser von weniger als 200 µm aufweist.

6. Die Zusammensetzung nach einem der Ansprüche 1 bis 3, wobei die Zusammensetzung innerhalb der ersten Minute nach einer Verabreichung in eine Verwendungsumgebung ein Molverhältnis von gelöstem Cyclodextrin zu gelöstem Arzneistoff, welches höher als eins ist, bereitstellt.

7. Die Zusammensetzung nach Anspruch 6, wobei das Molverhältnis des gelösten Cyclodextrins zu dem gelösten Arzneistoff mindestens 1,5 beträgt.

8. Die Zusammensetzung nach Anspruch 6, wobei das Molverhältnis des gelösten Cyclodextrins zu dem gelösten Arzneistoff mindestens 2 beträgt.

9. Die Zusammensetzung nach Anspruch 6, wobei das Molverhältnis des gelösten Cyclodextrins zu dem gelösten Arzneistoff mindestens 3 beträgt.

10. Die Zusammensetzung nach Anspruch 1 oder 3, wobei die lösungsretardierte Form eine gelöste Arzneistoffkonzentration etwa eine Minute nach einer Verabreichung in eine Verwendungsumgebung, welche niedriger als 80 % der Konzentration von gelöstem Arzneistoff ist, die durch eine Kontrollzusammensetzung bereitgestellt wird, welche aus kristallinem Arzneistoff besteht, bereitstellt.

11. Die Zusammensetzung nach Anspruch 2 oder 3, wobei die lösungsgesteigerte Form des Cyclodextrins mindestens 50 % ist, gelöst eine Minute nach einer Verabreichung in eine Verwendungsumgebung.

12. Die Zusammensetzung nach Anspruch 11, wobei das lösungsgesteigerte Cyclodextrin mindestens 60 % ist, gelöst eine Minute nach einer Verabreichung in die Verwendungsumgebung.

13. Die Zusammensetzung nach einem der Ansprüche 6, 10 und 11, wobei die Verwendungsumgebung eine in vitro-Testlösung, umfassend eine 0,05 M KH₂PO₄-Pufferlösung, ist.

14. Die Zusammensetzung nach einem der Ansprüche 1 bis 3, eingebracht in eine feste Dosierungsform.

15. Die Zusammensetzung nach Anspruch 14, wobei die feste Dosierungsform kaubar ist.

16. Die Zusammensetzung nach einem der Ansprüche 1 bis 3, wobei der Arzneistoff aus der Gruppe ausgewählt ist, welche aus Antazida, Analgetika, Mittel gegen Angina, Mittel gegen Angst, Antiarrhythmika, antibakteriellen Mitteln, Antibiotika, Antidiarrhoika, Antidepressiva, Antiepileptika, Antimykotika, Antihistaminika, Antihypertonika, entzündungshemmenden Mitteln, Virustatika, Herzmitteln, Kontrazeptiva, hustenhemmenden Mitteln, zytotoxischen Mitteln, Dekongestionsmitteln, Diuretika, Arzneistoffen für Urogenitalstörungen, Arzneistoffen zur Verwendung bei Parkinson-Krankheit und verwandten Störungen, Arzneistoffen zur Verwendung bei rheumatischen Störungen, Schlafmitteln, Mineralien und Vitaminen, lipidsenkenden Arzneistoffen und Geschlechtshormonen besteht.

17. Die Zusammensetzung nach Anspruch 16, wobei der Arzneistoff ein Antihistaminikum, umfassend ein Benzhydrylpiperazin, ist.

18. Die Zusammensetzung nach Anspruch 17, wobei der Arzneistoff Cetirizin ist.

## Revendications

1. Composition pharmaceutique comprenant un mélange physique de
(a) un médicament au goût déplaisant sous une forme solide ; et
(b) une cyclodextrine sous une forme solide ;
ledit médicament se trouvant sous une forme à dissolution retardée choisie parmi une forme multiparticulaire et un revêtement.

2. Composition pharmaceutique comprenant un mélange physique de
(a) un médicament au goût déplaisant sous une forme solide ; et
(b) une cyclodextrine sous une forme solide ;
ladite cyclodextrine étant sous une forme à dissolution améliorée choisie parmi une forme amorphe et une taille moyenne de particules inférieure à 125 µm.

3. Composition pharmaceutique comprenant un mélange physique de
(a) un médicament au goût déplaisant sous une forme solide ; et
(b) une cyclodextrine sous une forme solide ;
ledit médicament se trouvant sous une forme à dissolution retardée choisie parmi une forme multiparticulaire et un revêtement et ladite cyclodextrine étant sous une forme à dissolution améliorée choisie parmi une forme amorphe et une taille moyenne de particules inférieure à 125 µm.

4. Composition selon la revendication 1 ou 3, dans laquelle ladite forme multiparticulaire a un diamètre moyen inférieur à 500 µm.

5. Composition selon la revendication 1 ou 3, dans laquelle ladite forme multiparticulaire a un diamètre moyen inférieur à 200 µm.

6. Composition selon l'une quelconque des revendications 1 ou 3, dans laquelle ladite composition fournit à un environnement d'utilisation dans la minute suivant l'administration un rapport molaire de la cyclodextrine dissoute sur le médicament dissous qui est supérieur à un.

7. Composition selon la revendication 6, dans laquelle ledit rapport molaire de ladite cyclodextrine dissoute sur ledit médicament dissous est d'au moins 1,5.

8. Composition selon la revendication 6, dans laquelle ledit rapport molaire de ladite cyclodextrine dissoute sur ledit médicament dissous est d'au moins 2.

9. Composition selon la revendication 6, dans laquelle ledit rapport molaire de ladite cyclodextrine dissoute sur ledit médicament dissous est d'au moins 3.

10. Composition selon la revendication 1 ou 3, dans laquelle ladite forme à dissolution retardée fournit une concentration de médicament dissous à un environnement d'utilisation environ une minute après l'administration qui est inférieure à 80 % de la concentration en médicament dissous fournie par une composition témoin constituée par un médicament cristallin.

11. Composition selon la revendication 2 ou 3, dans laquelle ladite forme à dissolution améliorée de ladite cyclodextrine est dissoute à au moins 50 % une minute après l'administration à un environnement d'utilisation.

12. Composition selon la revendication 11, dans laquelle ladite cyclodextrine à dissolution améliorée est dissoute à au moins 60 % une minute après l'administration à l'environnement d'utilisation.

13. Composition selon l'une quelconque des revendications 6, 10 et 11, dans laquelle ledit environnement d'utilisation est une solution de test *in vitro* comprenant une solution tampon de KH₂PO₄ 0,05 M.

14. Composition selon l'une quelconque des revendications 1 à 3, incorporée dans une forme posologique solide.

15. Composition selon la revendication 14, dans laquelle ladite forme posologique solide est une forme à mâcher.

16. Composition selon l'une quelconque des revendications 1 à 3, dans laquelle ledit médicament est choisi dans le groupe constitué par les antiacides, les analgésiques, les antiangineux, les agents anti-anxiété, les anti-arythmiques, les antibactériens, les antibiotiques, des antidiarrhéiques, les antidépresseurs, les antiépileptiques, les antifongiques, les antihistaminiques, les antihypertenseurs, les agents anti-inflammatoires, les antiviraux, les agents cardiaques, les contraceptifs, les antitussifs, les cytotoxiques, les décongestionnants, les diurétiques, les médicaments destinés aux troubles génito-urinaires, les médicaments destinés à être utilisés dans le parkinsonisme et les troubles apparentés, les médicaments destinés à être utilisés dans les troubles rhumatismaux, les hypnotiques, les minéraux et les vitamines, les hypolipémiants et les hormones sexuelles.

17. Composition selon la revendication 16, dans laquelle ledit médicament est un antihistaminique qui comprend une benzhydrylpipérazine.

18. Composition selon la revendication 17, dans laquelle ledit médicament est la cétirizine.
